**Europäisches Patentamt**

**European Patent Office**

**Office européen des brevets**

(19)

(11) Numéro de publication : **0 346 196 B1**

(12)

# FASCICULE DE BREVET EUROPEEN

(45) Date de publication du fascicule du brevet :
**04.08.93 Bulletin 93/31**

(51) Int. Cl.⁵ : **C07C 59/265,** C07C 51/47

(21) Numéro de dépôt : **89401529.6**

(22) Date de dépôt : **02.06.89**

(54) **Procédé de récupération d'acide citrique à partir d'une liqueur en contenant.**

(30) Priorité : **06.06.88 FR 8807505**

(43) Date de publication de la demande :
**13.12.89 Bulletin 89/50**

(45) Mention de la délivrance du brevet :
**04.08.93 Bulletin 93/31**

(84) Etats contractants désignés :
**AT BE CH DE ES GB IT LI NL**

(56) Documents cités :
**FR-A- 1 280 940**
**FR-A- 1 290 212**
**US-A- 4 720 579**
**CHEMICAL ABSTRACTS, vol. 90, no. 21, 21 mai 1979, page 557, résumé no. 168073h, Columbus, Ohio, US; & JP-A-78 144 526**

(73) Titulaire : **Roquette Frères**
**F-62136 Lestrem (FR)**

(72) Inventeur : **Duflot, Pierrick**
**155 Rue des Mioches**
**F-62136 Lestrem (FR)**
Inventeur : **Leleu, Jean Bernard**
**22 Place du 8 mai**
**F-62136 Lestrem (FR)**

(74) Mandataire : **Koch, Gustave et al**
**Cabinet PLASSERAUD 84, rue d'Amsterdam**
**F-75009 Paris (FR)**

EP 0 346 196 B1

## Description

L'invention a pour objet un procédé de récupération d'acide citrique à partir d'une liqueur en contenant.

On désigne par le terme "liqueur" les liqueurs de fermentation et les eaux-mères de fermentation de l'acide citrique.

Il est connu de préparer de l'acide citrique par cristallisation à partir de sirops suffisamment riches en ce produit et suffisamment concentrés.

Il est également connu de préparer les sirops en question à partir de liqueurs de fermentation.

Ainsi, il est usuel de traiter à la chaux des liqueurs de fermentation préalablement débarrassées du mycélium et de récupérer par filtration le citrate de calcium insoluble ainsi formé, ce grâce à quoi en élimine toutes les impuretés solubles; ce citrate de calcium est décomposé par l'acide sulfurique de façon à libérer l'acide citrique. Le sulfate de calcium qui se forme au cours de cette réaction est séparé par filtration; les cations résiduels sont éliminés par passage du filtrat contenant l'acide citrique sur une résine échangeuse d'ions fonctionnant dans le cycle hydrogène dans des conditions telles que seuls les cations sont adsorbés.

Ce procédé qui permet d'éliminer les impuretés minérales et organiques solubles présentes dans les liqueurs de fermentation et qui fournit avec un bon rendement des solutions suffisamment riches en acide citrique pour faire cristalliser ce dernier, présente les inconvénients majeurs d'être gros consommateur d'acide sulfurique et gros générateur de sulfate de calcium, qui est un produit polluant, ni recyclable, ni réutilisable.

Pour éviter ces inconvénients, on a essayé de mettre au point d'autres procédés qui permettent de se débarrasser partiellement des impuretés contenues dans les liqueurs de fermentation d'acide citrique.

La plupart de ces procédés sont basés sur l'extraction par des solvants organiques de l'acide citrique contenu dans ces liqueurs. Pour des raisons diverses, ils n'ont par donné entièrement satisfaction et n'ont jamais abouti à une exploitation industrielle.

D'autres procédés de purification, propres à séparer la majeure partie des impuretés constituées par des molécules de poids moléculaire élevé, sont basés sur l'utilisation

- soit de membranes filtrantes d'osmose inverse ou d'ultrafiltration qui excluent ces molécules de poids moléculaire élevé,
- soit de résines adsorbantes non ioniques qui les adsorbent.

Toutefois, les résines adsorbantes doivent être régénérées après chaque cycle d'adsorption à l'aide d'alcalis et de solvants organiques.

De plus, dans l'un et l'autre cas, les impuretés ioniques de faible poids moléculaire, qui n'ont pas été séparées de l'acide citrique et qui consistent surtout en cations et anions minéraux, sont éliminées par passage des liqueurs sur des résines cationiques et anioniques dans des conditions telles que, dans le cas des premières, seuls les cations et, dans le cas des secondes, seuls les anions d'acides forts minéraux sont adsorbés, réalisant ainsi une purification par échange d'ions. Lorsque ces résines sont saturées, les impuretés adsorbées sont éluées, lorsqu'il s'agit de la résine cationique à l'aide d'une solution d'acide fort, généralement de l'acide chlorhydrique et, lorsqu'il s'agit de la résine anionique à l'aide d'une solution de base forte, généralement de la soude, de façon à les régénérer et les rendre aptes à un nouveau cycle de purification par échange d'ions.

Ces procédés sont donc extrêmement coûteux et délicats à mettre en oeuvre et n'ont pas non plus connu de développement à l'échelle industrielle.

Le brevet FR-A-1 290 212 décrit un procédé de décoloration et de purification de solutions impures d'acides organiques, dont l'acide citrique, suivant lequel lesdites solutions, obtenues par fermentation, sont mises en contact avec un échangeur de cations possèdant des groupements actifs acides forts.

Suivant ce procédé, ce sont les impuretés contenues dans les susdites solutions qui sont retenues sur la résine.

Le document Chemical Abstracts, vol. 90, No. 21, 21 mai 19790, page 557, résumé 168073h, décrit, quant à lui, la récupération de l'acide tartrique à partir d'une solution aqueuse d'un de ses sels, à savoir le ditartrate de Na, par mise en contact avec un échangeur de cations, ce procédé s'appliquant aussi à l'acide citrique.

Aucune des techniques connues ne donnant par conséquent entièrement satisfaction, la Société Demanderesse s'est donné pour but de développer un procédé propre à permettre la récupération de l'acide citrique à partir de liqueurs de fermentation ou d'eaux-mères de cristallisation en contenant, sans recourir à l'acide sulfurique ou à des solvants organiques, tout en étant simple et économique à mettre en oeuvre.

Et le procédé qu'elle a eu le mérite de mettre au point est caractérisé par le fait que, selon la revendication 1 successivement,

- dans une première phase, la liqueur contenant de l'acide citrique est mise au contact d'une résine cationique sous forme hydrogène durant un temps suffisant pour atteindre une adsorption optimale de l'acide citrique,
- dans une seconde phase, la résine est traitée par un agent d'élution et la fraction d'éluat riche en acide

EP 0 346 196 B1

citrique purifié est récupérée.

La matière première soumise à la fermentation citrique est généralement constituée par des mélasses mais il est également possible d'avoir recours à des hydrates de carbone purs, auquel cas la fermentation citrique permet d'obtenir des liqueurs suffisamment pauvres en impuretés pour permettre au moins une première cristallisation d'acide citrique sans purification, seules les eaux-mères de cristallisation étant alors traitées conformément à l'invention de façon à extraire un deuxième ou un troisième jet d'acide cristallisé.

De préférence, la liqueur mise au contact de la résine est chaude, sa température étant avantageusement de 50 à 95°C et plus préférentiellement comprise entre 65 et 80°C.

Selon un mode de réalisation avantageux du procédé conforme a l'invention, l'agent d'élution est constitué d'eau; la température de cette eau est supérieure à 40°C, de préférence de 50 à 95°C et, plus préférentiellement encore, voisine de celle de la liqueur traitée.

Selon un autre mode de réalisation avantageux du procédé conforme à l'invention, la résine mise en oeuvre est à faible granulométrie et à faible taux de réticulation au divinylbenzène, les résines cationiques du type styrène-divinylbenzène étant préférées.

Le taux de réticulation de ces résines est généralement compris entre 5 et 10%, de préférence entre 4 et 8%, et leur granulométrie est comprise entre 0,1 à 1 mm, de préférence entre 0,2 et 0,6 mm.

Pour obtenir selon la revendication 1 une adsorption optimale de l'acide citrique, c'est-à-dire une adsorption d'une proportion d'au moins environ 90% de la quantité totale d'acide citrique contenu dans la liqueur, une fraction donnée de liqueur doit rester au contact de la résine de 20 min à 10 h, de préférence de 1 h à 3 h 1/2; dans la pratique, cette durée correspond à un débit horaire de 0,1 volume à 3 volumes, de préférence de 0,3 volume à 1 volume par volume de résine.

L'invention vise encore d'autres dispositions dont il sera question ci-après et elle pourra, de toute façon, être bien comprise à l'aide du complément de description qui suit et des exemples non limitatifs relatifs à des modes de réalisation avantageux, lesdits complément de description et exemples étant illustrés par les dessins dans lesquels

- la figure 1 est une représentation schématique d'une installation propre à la réalisation du procédé conforme à l'invention et
- les figures 2 et 3 montrent des courbes représentatives du déroulement du susdit procédé.

La liqueur de fermentation ou les eaux-mères de cristallisation dont la teneur en matières sèches est généralement de 10 à 75% en poids et, en particulier, voisine de 50% en poids et qui contient en grande partie l'acide citrique sous sa forme libre, est mise tout d'abord au contact d'une résine cationique sous forme hydrogène dans des conditions telles que tous les cations de la matière première soient remplacés par des protons. Cette permutation, bien qu'elle ne soit pas obligatoire, a l'avantage de protéger les évaporateurs de la formation de dépôts incrustants et assure une plus grande longévité aux résines d'adsorption.

Ensuite, on met la liqueur à purifier au contact de la résine sélectionnée, qui peut être par exemple l'une de celles identifiées ci-après par leur appellation commerciale:

- TSW 40      commercialisée par BAYER,
- C204      commercialisée par DUOLITE,
- DIAION SK1BS      commercialisée par MITSUBISHI,
- CFX4      commercialisée par SYBRON.

A cet effet, on dispose avantageusement la résine dans des colonnes chemisées thermostatées. Ce chemisage des colonnes n'est pas nécessaire lorsque les dimensions de l'installation sont telles que les pertes thermiques ne présentent plus un inconvénient majeur.

La durée de contact est celle nécessaire pour réaliser une adsorption optimale de l'acide citrique.

Ensuite, on élue la résine. Pour ce faire, on peut avoir recours à de l'eau de préférence chaude, d'une température voisine de celle de la matière première; en général, cette température est comprise entre 50 et 95°C.

Dans la pratique, le débit d'élution est de 0,1 volume à 3 volumes, de préférence de 0,3 volume à 1 volume, d'agent d'élution par volume de résine et par heure.

On recueille les fractions éluées suivant des parties aliquotes.

On constate que, de façon inattendue et tout à fait surprenante,

- les premières fractions éluées contiennent essentiellement des produits de forte masse moléculaire ainsi que des anions minéraux et des sucres qui n'ont pas été fermentés, à savoir notamment du saccharose, maltose, isomaltose et autres,
- les fractions suivantes contiennent l'acide citrique à une pureté très élevée et
- les dernières fractions contiennent divers acides organiques tels que les acides gluconique, oxalique et autres ainsi que d'autres composés s'adsorbant très fortement sur la résine tels que la bétaïne, composés qui ont été apportés par les éléments constitutifs du milieu de culture ou synthétisés au cours de la fermentation en même temps que l'acide citrique.

3

Les fractions contenant l'acide citrique sont concentrées à une teneur en matières sèches suffisante pour que l'acide citrique cristallise, c'est-à-dire supérieure à environ 60% en poids, l'acide citrique cristallisé étant ensuite séparé par filtration.

Le procédé conforme à l'invention conduit à une séparation quasi-parfaite de l'acide citrique des impuretés contenues dans les liqueurs sans consommation de produits chimiques ni formation de produits secondaires polluants.

Pour la mise en oeuvre du procédé conforme à l'invention, on peut avoir recours à une ou plusieurs colonnes de chromatographie disposées en série ou en parallèle et fonctionnant de façon continue ou discontinue; ces colonnes doivent être équipées de moyens propres à les alimenter en matière première et en agent d'élution et elles doivent également être équipées de moyens propres à collecter les fractions d'éluat successives.

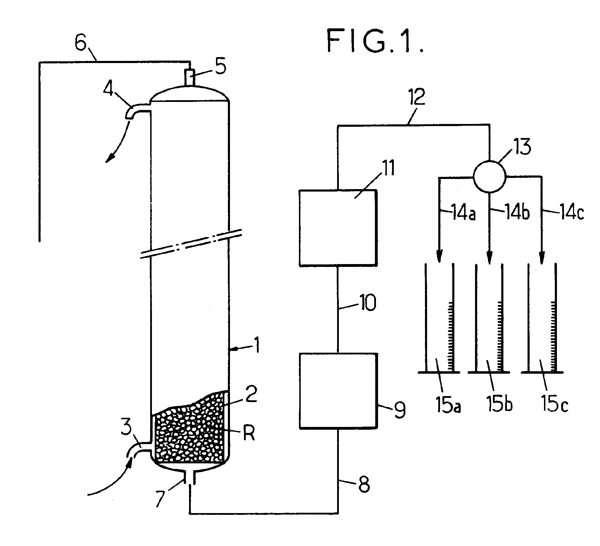

A titre illustratif, on a montré figure 1 une installation à une seule colonne fonctionnant de manière discontinue. Des performances nettement supérieures peuvent être réalisées à l'aide d'installations à plusieurs colonnes fonctionnant de façon continue, notamment du type de celle décrite dans le brevet français N° 79 10564 de la Demanderesse; les performances des installations en question sont représentées par la quantité de matière sèche de liqueur purifiée par volume de résine et par unité de temps ainsi que par la teneur en matières sèches des diverses fractions contenant l'acide citrique purifié, d'une part, et les impuretés, d'autre part; ceci étant, il est à noter que la richesse des diverses fractions séparées est identique, quelle que soit l'installation utilisée.

L'installation réduite à une colonne unique de la figure 1 comprend une colonne 1 de verre à double enveloppe 2 d'une capacité de 350 ml, d'un diamètre d'environ 1,5 cm et d'une hauteur de 2 m; elle est chargée de 339 ml de résine cationique forte, montrée en R, par exemple celle vendue sous la dénomination "C204" par la Société DUOLITE, réticulée à 8% de divinylbenzène. La double enveloppe de la colonne est alimentée en eau thermostatée par l'embout 3, cette eau étant évacuée par l'embout 4 de façon à ce que la température de la résine soit maintenue à 75°C.

La résine est mise sous sa forme acide ou hydrogène par percolation de 1000 ml d'une solution aqueuse 1,2 N d'acide chlorhydrique; pour cette percolation, l'acide chlorhydrique est amené au sommet de la colonne à un embout 5 par une canalisation 6; par la même canalisation, on amène ensuite de l'eau de rinçage. La percolation et le rinçage s'effectuent à un débit d'environ 2 volumes de liquide par volume de résine et par heure. On utilise pour le rinçage 2000 ml d'eau. Les liquides sortent de la colonne par un embout 7.

L'embout 7 est relié par une canalisation 8 à un réfractomètre différentiel 9 permettant de mesurer l'indice de réfraction du liquide sortant de la colonne et, par là même, de détecter et de quantifier la présence de matières solubles, ce réfractomètre étant lui-même relié par une canalisation 10 à un détecteur UV montré en 11, fonctionnant à 280 nm de façon à détecter les matières azotées; le réfractomètre 9 et le détecteur 11 sont disposés en série.

Le détecteur 11 est relié par une canalisation 12 à un robinet 13 à quatre voies, propre à diriger par des canalisations 14a, 14b et 14c l'eluat acheminé par la canalisation 12 sur trois récipients gradués respectivement 15a, 15b et 15c.

Après permutation sous forme acide de la résine contenue dans la colonne, on injecte au sommet de celle-ci une certaine quantité de liqueur de fermentation, puis on élue cette liqueur par de l'eau chaude.

Les indications fournies par le détecteur UV 11 et le réfractomètre 9 permettent de séparer l'éluat en sortie de colonne en trois fractions contenant respectivement:
- les impuretés exclues,
- l'acide citrique purifié,
- les impuretés adsorbées

ces trois fractions étant introduites respectivement dans les récipients 15a, 15b et 15c.

## EXEMPLE 1

Récupération d'acide citrique à partir d'une liqueur de fermentation obtenue par fermentation d'un milieu à base d'hydrolysat d'amidon.

La liqueur traitée présente une richesse en acide citrique de 89,3% en poids par rapport à la matière sèche et contient des impuretés organiques et minérales dont 1,1% d'acide gluconique, 0,5% d'acide oxalique, 2% de maltose et environ 2% de cendres sulfatées; elle a été filtrée et concentrée à une teneur en matières sèches de 50%; sa densité est de 1,22 g/ml.

Une quantité de 1 ml de cette liqueur est introduite au sommet de la colonne de résine décrite ci-dessus et illustrée par la figure 1.

L'élution est effectuée au moyen d'eau chaude à un débit de 152 ml/heure; la température de la double enveloppe est maintenue à 75°C.

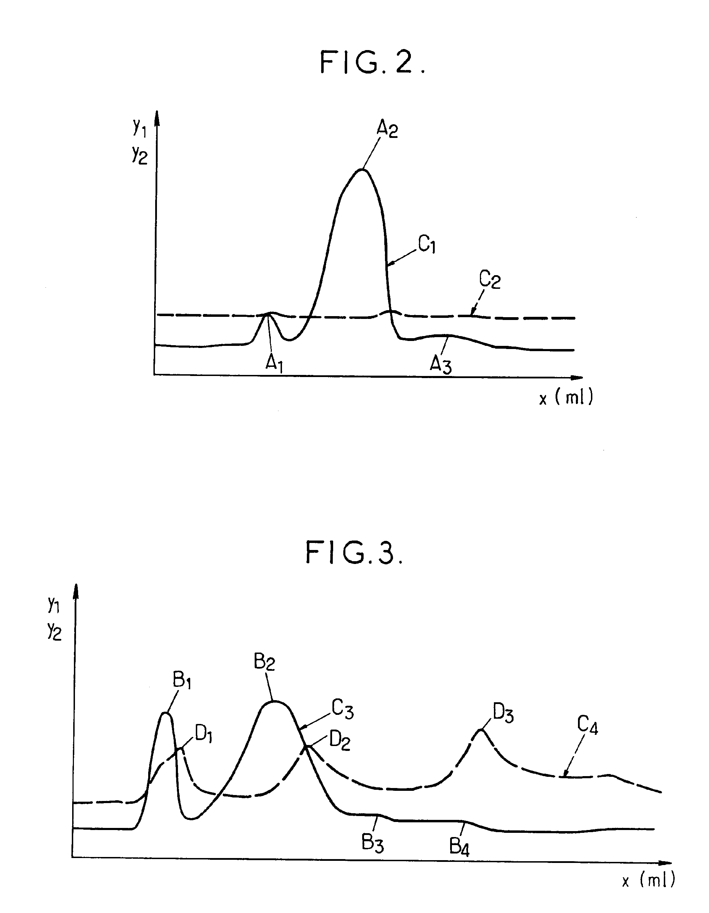

L'éluat commence à apparaître à la sortie de la colonne après environ 42 minutes. La variation de sa teneur en matières sèches en fonction de la quantité x, exprimée en ml, d'eluat recueilli, est matérialisée par une courbe $C_1$ représentant sur le diagramme de la figure 2 la variation y1 (échelle arbitraire) de la lecture réfractométrique (réfractomètre 9) en fonction de x.

La courbe $C_1$ comporte 3 pics repérés par les lettres $A_1$, $A_2$ et $A_3$.

Le pic $A_1$ correspond à une première fraction contenant les impuretés exclues et représente un volume de 16 ml. Cette première fraction d'eluat est recueillie dans le récipient 15a vers lequel elle est acheminée par une canalisation 14a grâce au robinet 13.

Le pic $A_2$ représente un volume de 43 ml et correspond à une deuxième fraction qui contient l'acide citrique purifié, fraction qui est recueillie dans le récipient 15b par une manoeuvre du robinet 13 acheminant cette fraction par la canalisation 14b.

Le pic $A_3$ représente un volume de 36 ml et correspond à une troisième fraction qui contient des impuretés plus fortement adsorbées que l'acide citrique; cette fraction est recueillie dans le récipient 15c par une nouvelle manoeuvre du robinet 13 acheminant l'éluat par la canalisation 14c.

La variation y2 de la densité optique (échelle arbitraire) à 280 nm de l'éluat, toujours en fonction de la quantité x d'éluat recueilli, mesurée par le détecteur 11, est matérialisée par une courbe $C_2$ montrée sur le diagramme de la figure 2.

La courbe $C_2$ est pratiquement plate durant toute la durée de l'élution et n'apporte pas, dans ce cas, d'éléments permettant de juger de l'efficacité de la purification. En effet, les matières premières composant les liqueurs de fermentation étaient très pures (hydrolysat d'amidon) et ne comportaient pas de substances absorbant le rayonnement ultraviolet.

Dans le tableau I, on a réuni les valeurs de la teneur totale en matières sèches et de la proportion d'acide citrique pour les trois fractions correspondant aux pics $A_1$, $A_2$ et $A_3$.

## TABLEAU I

| | Teneur totale en matières sèches (en mg) | Proportion d'acide citrique (en %) |
|---|---|---|
| Fraction correspondant au pic $A_1$ | 31 | 5 |
| Fraction correspondant au pic $A_2$ | 534 | 99 |
| Fraction correspondant au pic $A_3$ | 45 | 5 |

Il résulte de ces valeurs que la fraction B correspondant au pic $A_2$ contient 97% de l'acide citrique présent dans la liqueur soumise à la purification, sa richesse en acide citrique étant de 99%.

## EXEMPLE 2

Récupération d'acide citrique à partir d'une liqueur de fermentation obtenue par fermentation d'un milieu à base de mélasses.

La liqueur traitée présente une richesse en acide citrique de 63,5% en poids par rapport à la matière sèche et contient des impuretés organiques et minérales dont 6,5% d'acide gluconique, 0,3% d'acide oxalique, 1,2% de fructose, 3,5% de glucose, 6,6% de disaccharides et 13,5% de cendres sulfatées; elle a été filtrée et présente une teneur en matières sèches de 16,5%; sa densité est de 1,07 g/ml.

Une quantité de 2 ml de cette liqueur est introduite au sommet de la colonne de résine décrite ci-dessus et illustrée par la figure 1.

L'élution est effectuée au moyen d'eau chaude à un débit de 162 ml/heure; la température de la double enveloppe est maintenue à 75°C.

L'éluat commence à apparaître à la sortie de la colonne après environ 42 minutes. La variation de sa teneur

5

en matières sèches en fonction de la quantité x, exprimée en ml d'éluat recueilli, est matérialisée par une courbe $C_3$ représentant sur le diagramme de la figure 3 la variation y1 (échelle arbitraire) de la lecture réfractométrique (réfractomètre 9) en fonction de x.

La courbe C3 comporte 4 pics repérés par les lettres $B_1$, $B_2$, $B_3$ et $B_4$.

Le pic $B_1$ correspond à une première fraction contenant les impuretés exclues et représente un volume de 22 ml. Cette première fraction d'éluat est recueillie dans le récipient 15a vers lequel elle est acheminée par une canalisation 14a grâce au robinet 13.

Le pic $B_2$ représente un volume de 59 ml et correspond à une deuxième fraction qui contient l'acide citrique purifié, fraction qui est recueillie dans le récipient 15b par une manoeuvre du robinet 13 acheminant cette fraction par la canalisation 14b.

Les pics $B_3$ et $B_4$ représentent un volume de 54 ml et correspondent à une troisième fraction qui contient des impuretés plus fortement adsorbées que l'acide citrique; cette fraction est recueillie dans le récipient 15c par une nouvelle manoeuvre du robinet 13 acheminant l'éluat par la canalisation 14c.

La variation y2 de la densité optique (échelle arbitraire) à 280 nm de l'éluat, toujours en fonction de la quantité x d'éluat recueilli, mesurée par le détecteur 11, est représentée par la courbe $C_4$ sur le diagramme de la figure 3.

Cette courbe comporte trois pics $D_1$, $D_2$ et $D_3$ représentatifs des impuretés contenues dans les mélasses et on constate que ces impuretés, révélées par les pics $D_1$ et $D_3$, se retrouvent en totalité dans les fractions $B_1$ et $B_3$ + $B_4$ ainsi qu'une partie des impuretés dont la présence est révélée par le pic $D_2$.

Il s'ensuit que même sur des liqueurs dont la teneur en acide citrique était relativement basse, le procédé selon l'invention permet d'obtenir l'acide citrique avec une grande pureté et un excellent rendement. Les valeurs trouvées pour la teneur totale en matières sèches et la proportion d'acide citrique dans le cas des trois fractions, sont réunies dans le tableau II.

## TABLEAU II

| | Teneur totale en matières sèches (en mg) | Proportion d'acide citrique (en %) |
|---|---|---|
| Fraction correspondant au pic $B_1$ | 84 | 8 |
| Fraction correspondant au pic $B_2$ | 215 | 98 |
| Fraction correspondant aux pics $B_3$+$B_4$ | 54 | 13 |

Il apparaît à l'examen du Tableau II que la fraction correspondant au pic $B_2$ contient 94% de l'acide citrique présent dans la liqueur soumise à la purification; la richesse de cette fraction en acide citrique est de 98%.

En suite de quoi on dispose ainsi d'un procédé de récupération d'acide citrique à partir de liqueurs de fermentation, procédé dont les caractéristiques résultent suffisamment de ce qui précède pour qu'il soit inutile d'insister à ce sujet et qui présente, par rapport à ceux qui existent déjà, de nombreux avantages dont

- celui de permettre de récupérer de l'acide citrique hautement purifié par un traitement chromatographique unique d'une liqueur de fermentation d'acide citrique ou des eaux-mères d'une telle liqueur dont on a déjà séparé par cristallisation un premier jet dudit acide,
- celui de ne pas consommer de réactifs chimiques,
- celui de ne pas générer de produits polluants,
- celui de mette en oeuvre des matériaux (résines) dont l'innocuité et la stabilité sont reconnues,
- celui de pouvoir être mis en oeuvre dans des installations dont la robustesse et la fiabilité ont été prouvées par l'usage intensif qui en a été fait dans d'autres applications.

**Revendications**

1. Procédé de récupération d'acide citrique à partir d'une liqueur en contenant, caractérisé par le fait que, successivement,
   - dans une première phase, la liqueur contenant de l'acide citrique est mise au contact d'une résine cationique sous forme hydrogène pendant une durée de 20 min à 10 h, de préférence de 1 h à 3 h 1/2, ce qui correspond à un débit horaire de 0,1 volume à 3 volumes, de préférence de 0,3 volume à 1 volume par volume de résine,
   - dans une seconde phase, la résine est traitée par un agent d'élution et la fraction d'éluat riche en acide citrique purifié est récupérée.

2. Procédé selon la revendication 1, caractérisé par le fait que l'agent d'élution est constitué d'eau.

3. Procédé selon la revendication 2, caractérisé par le fait que l'eau a une température supérieure à 40°C et, de préférence, compriser entre 50 et 95°C.

4. Procédé selon l'une des revendications 1 à 3, caractérisé par le fait que la température de la liqueur mise au contact de la résine est de 50 à 95°C, de préférence de 65 à 80°C et que la température de l'agent d'élution est de préférence voisine de celle de la liqueur.

5. Procédé selon l'une des revendications 1 à 4, caractérisé par le fait que la résine mise en oeuvre est à faible granulométrie et à faible taux de réticulation au divinylbenzène, les résines cationiques du type styrènedivinylbenzène étant préférées, le taux de réticulation de ces résines étant généralement compris entre 5 et 10%, de préférence entre 4 et 8%, et leur granulomérie comprise entre 0,1 et 1 mm, de préférence entre 0,2 et 0,6 mm.

6. Procédé selon l'une des revendications 1 à 5, caractérisé par le fait que la liqueur est obtenue à partir de mélasses ou d'hydrates de carbone.


**Patentansprüche**

1. Verfahren zur Gewinnung von Citronensäure aus einer diese enthaltenden Flüssigkeit, dadurch gekennzeichnet, daß nacheinander
   - in einer ersten Phase die die Citronensäure enthaltende Flüssigkeit mit einem kationischen Harz in der Wasserstoff-Form während einer Dauer von 20 Minuten bis zu 10 Stunden, vorzugsweise einer Stunde bis 3 1/2 Stunden, entsprechend einem stündlichen Durchsatz von 0,1 Volumen bis 3 Volumen, vorzugsweise 0,3 Volumen bis 1 Volumen, je Volumen Harz in Kontakt gebracht wird,
   - in einer zweiten Phase das Harz mit einem Elutionsmittel behandelt und die an gereinigter Citronensäure reiche Eluatfraktion gewonnen wird.

2. Verfahren gemäß Anspruch 1, dadurch gekennzeichnet, daß das Elutionsmittel aus Wasser besteht.

3. Verfahren gemäß Anspruch 2, dadurch gekennzeichnet, daß das Wasser eine Temperatur von höher als 40°C und vorzugsweise zwischen 50 und 95°C besitzt.

4. Verfahren gemäß einem der Ansprüche 1 bis 3, dadurch gekennzeichnet, daß die Temperatur der mit dem Harz in Kontakt gebrachten Flüssigkeit 50 bis 95°C, vorzugsweise 65 bis 80°C, beträgt und daß die Temperatur des Elutionsmittels vorzugsweise in der Nähe derjenigen der Flüssigkeit liegt.

5. Verfahren gemäß einem der Ansprüche 1 bis 4, dadurch gekennzeichnet, daß das eingesetzte Harz eine geringe Korngröße und einen geringen Vernetzungsgrad mit Divinylbenzol besitzt, wobei die kationischen Harze vom Styrol-Divinylbenzol-Typ bevorzugt sind, der Vernetzungsgrad dieser Harze im allgemeinen zwischen 5 und 10%, vorzugsweise zwischen 4 und 8%, liegt und ihre Korngröße zwischen 0,1 und 1 mm, vorzugsweise zwischen 0,2 und 0,6 mm, beträgt.

6. Verfahren gemäß einem der Ansprüche 1 bis 5, dadurch gekennzeichnet, daß die Flüssigkeit aus Melassen oder aus Kohlenhydraten erhalten wird.

## Claims

1. Method for the recovery of citric acid from a liquor containing the same, characterized by the fact that, successively,
    - in a first step, the liquor containing citric acid is put into contact with a cationic resin in the hydrogen form during a duration from 20 minutes to 10 hours, preferably from 1 h to 3 h 1/2, which corresponds to a delivery per hour of 0.1 volume to 3 volumes, preferably from 0.3 volume to 1 volume per volume of resin,
    - in a second step, the resin is treated by means of an elution agent and the fraction of eluate rich in purified citric acid is recovered.

2. Method according to claim 1, characterized by the fact that the elution agent is consisting of water.

3. Method according to claim 2, characterized by the fact that water has a temperature higher than 40°C and preferably comprised between 50 and 95°C.

4. Method according to one of claims 1 to 3, characterized by the fact that the temperature of the liquor put into contact with the resin is from 50 to 95°C, preferably from 65 to 80°C and that the temperature of the elution agent is preferably close to the temperature of the liquor.

5. Method according to one of claims 1 to 4, characterized by the fact that the resin which is implemented has a low granulometry and a low rate of reticulation with divinylbenzene, cationic resins of the styrenedivinylbenzene type being preferred, the rate of reticulation of the said resins being generally comprised between 5 and 10%, preferably between 4 and 8%, and their granulometry being comprised between 0.1 and 1 mm, preferably between 0.2 and 0.6 mm.

6. Method according to claims 1 to 5, characterized by the fact that the liquor is obtained from molasses or from carbon hydrates.

# FIG.1.

# FIG. 2.

# FIG.3.